Europäisches Patentamt

European Patent Office

Office européen des brevets

(19)

(11) Numéro de publication : **0 349 378 B1**

(12) **FASCICULE DE BREVET EUROPEEN**

(45) Date de publication du fascicule du brevet :
20.05.92 Bulletin 92/21

(51) Int. Cl.⁵ : **C07C 2/08**

(21) Numéro de dépôt : **89401728.4**

(22) Date de dépôt : **19.06.89**

(54) **Procédé catalytique de dimérisation, de codimérisation ou d'oligomérisation d'oléfines avec utilisation d'un fluide autogènede thermorégulation.**

(30) Priorité : **28.06.88 FR 8808799**

(43) Date de publication de la demande :
**03.01.90 Bulletin 90/01**

(45) Mention de la délivrance du brevet :
**20.05.92 Bulletin 92/21**

(84) Etats contractants désignés :
**CH DE GB IT LI NL**

(56) Documents cités :
**US-A- 2 222 304**
**US-A- 4 709 111**

(73) Titulaire : **INSTITUT FRANCAIS DU PETROLE**
**4, Avenue de Bois Préau**
**F-92502 Rueil-Malmaison (FR)**

(72) Inventeur : **Dang Vu, Quang**
**48 bis, Boulevard du Général Leclerc**
**F-92200 Neuilly (FR)**
Inventeur : **Chauvin, Yves**
**64, avenue du Général Leclerc**
**F-78230 Le Pecq (FR)**

EP 0 349 378 B1

**Description**

L'invention concerne un procédé pour effectuer, généralement sous pression, des réactions de dimérisation, de codimérisation et d'oligomérisation (sélectives notamment) d'oléfines en présence d'au moins un catalyseur, habituellement solide, dans au moins une zone réactionnelle dont la température est contrôlée par un dispositif d'échange thermique à base de plaques creuses disposé en son sein.

Généralement dans ce type de réactions, lorsqu'on opère selon l'invention, au moins un des réactifs se trouve soit à l'état liquide, soit dans un état rendant possible sa circulation par une pompe (état supercritique), tel que le rapport $T_R$ entre la température T (en kelvin) du système des réactifs et la température (pseudo-) critique $T_C$ (en kelvin) dudit système soit de préférence inférieur à 2, par exemple à 1,5.

L'oléfine employée peut notamment être choisie parmi l'éthylène, le propylène, le styrène, l'un des isomères des butènes, ou un de leurs mélanges. Cette oléfine peut être mise en oeuvre à l'état pur ou en mélange avec un ou plusieurs composés ne réagissant pas sur le catalyseur dans les conditions utilisées, tels que par exemple les hydrocarbures saturés cycliques ou acycliques, en particulier ceux ayant de 2 à 10 atomes de carbone. La concentration de l'oléfine dans le mélange peut être de 5 à 100 %, de préférence de 10 à 100 % en poids.

L'invention concerne plus particulièrement un procédé de dimérisation sélective du propylène en méthyl-4 pentène-1, par exemple au moyen de catalyseurs solides à base de potassium et/ou de sodium.

Il est connu de dimériser ou d'oligomériser des oléfines en phase liquide homogène pour obtenir, par exemple, des dimates en $C_6$ (brevets de la demanderesse US-A-4 283 305 et 4 366 087).

Il est beaucoup plus difficile de faire la dimérisation sélective quand on met en oeuvre un catalyseur hétérogène, par exemple pour dimériser le propylène en méthyl-4 pentène-1, avec une sélectivité suffisante, notamment supérieure à 85 %. Il faut en effet alors maintenir, dans des limites assez étroites, une température relativement constante, en-dessous de laquelle l'activité du catalyseur tombe à une valeur qui rend la réaction inexploitable industriellement et au-dessus de laquelle interviennent des réactions consécutives d'isomérisation qui abaissent la sélectivité et entraînent des problèmes de séparation quasi-insurmontables.

Le système réactionnel isotherme le plus souvent utilisé est le réacteur à tubes calandre monopasse où l'intérieur des tubes est rempli de catalyseur et constitue ainsi le milieu réactionnel. Mais, en dimérisation sélective, le système catalytique subit en général une désactivation spontanée et/ou accidentelle (due aux impuretés), ce qui nécessite le renouvellement périodique de ce catalyseur, contrainte techniquement difficile à mettre en oeuvre avec un réacteur dans lequel les tubes en nombre important doivent être remplis et vidés un à un de façon manuelle.

Il est également connu, lorsque la température de la réaction doit être maintenue dans des limites relativement étroites, de placer au sein du lit catalytique un appareil de transfert thermique soit à base de tubes (GB-B-2 046 618), soit à base de plaques (US-A-3 666 423), soit à base de grilles (US-A-4 693 807), et de faire circuler à l'intérieur de cet appareil un fluide destiné au transfert thermique et communément désigné sous le nom de fluide de thermorégulation.

L'inconvénient dans l'utilisation d'un appareil de transfert thermique à base de tubes provient du fait que la liaison entre ces tubes individuels est très encombrante et que, par conséquent, le montage de l'ensemble est très difficile à réaliser correctement a l'intérieur du réacteur. L'inconvénient de l'appareil de transfert thermique à base de plaques du brevet US-A-3 666 423 est son encombrement et sa faible efficacité. Pour pouvoir résister à la pression réactionnelle, les plaques ne sont que partiellement évidées et le fluide de thermorégulation ne dispose ainsi que d'une faible partie de la surface des plaques pour faire son travail d'échange.

Dans son brevet US-A-4 544 544, la demanderesse a proposé, pour des systèmes à réactifs gazeux, un procédé permettant d'utiliser des la plaques creuses, en tôles minces et à section interne rectangulaire.

Les plaques utilisées dans le procédé selon la présente invention travaillent très peu à la contrainte, ce qui permet de les évider totalement et de laisser le fluide de thermorégulation assurer l'échange à travers la totalité de la surface disponible. De plus, le montage et les connexions sont suffisamment simples pour être réalisés facilement dans l'espace restreint offert par le réacteur.

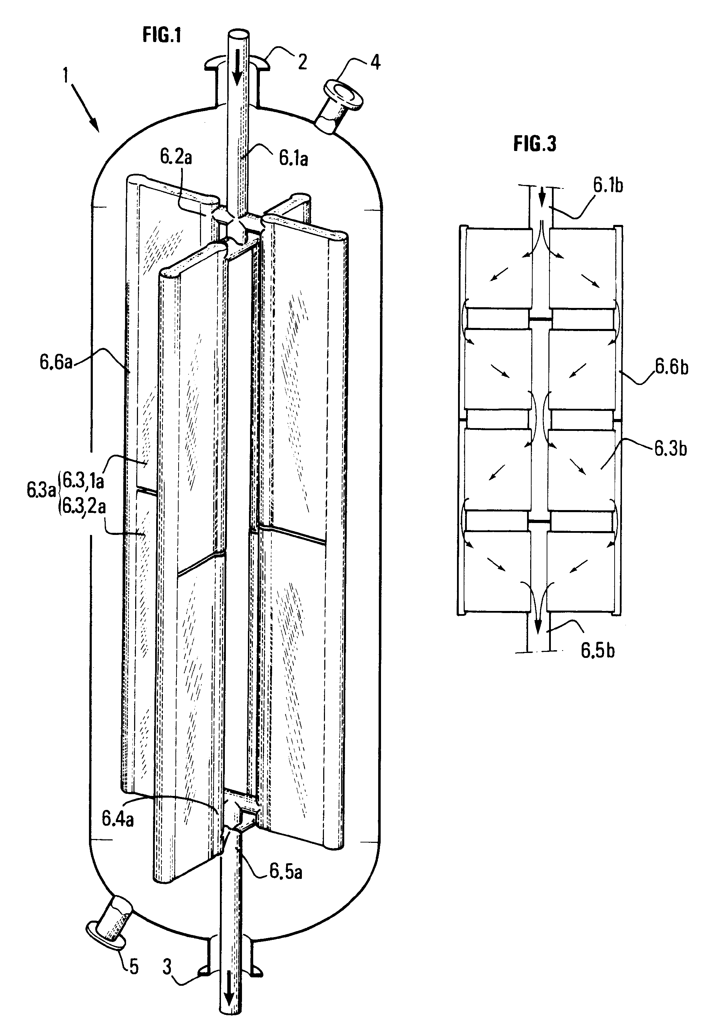

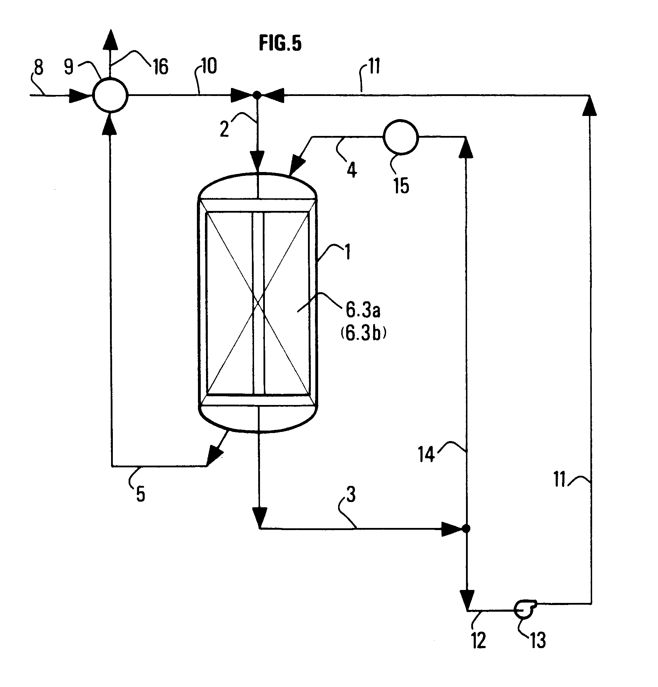

La présente invention utilise un système réactionnel mono-étagé continu, équipé d'un appareil à plaques (ou espaces internes creux) de transfert thermique. Les figures 1, 2 et 3 illustrent le cheminement du fluide de thermorégulation dans lesdits espaces internes creux et la figure 5 explique la marche de l'unité :

- le fluide réactionnel va d'abord être employé comme fluide de thermorégulation de l'unité et du lit de catalyseur ensuite, il traversera le lit de catalyseur solide enfermé dans une enveloppe (1) de forme sensiblement cylindrique et allongée ;
- le sein du lit catalytique est refroidi par le cheminement du fluide de thermorégulation qui circule dans un dispositif d'échange thermique plongée dans ledit lit catalytique et à base de plaques creuses fabriquées, par exemple, à partir de tôles minces plates ou ondulées, comme il sera expliqué plus loin ;

– le fluide de thermorégulation circulant à l'intérieur des plaques comprend le(s) réactif(s) constituant la charge fraîche, la circulation de ce fluide étant assurée habituellement par au moins une pompe (13) ;

– le système de thermorégulation est un système ouvert sur le système réactionnel : en effet, il est alimenté en continu par le(s) réactif(s) d'appoint, qui passe(nt) directement du système de thermorégulation dans le réacteur sans barrière mécanique permanente ou transitoire.

Selon le procédé de l'invention, on envoie la charge fraîche (par exemple du propylène liquide), sous une pression généralement comprise entre 1,2 et 12 MPa, dans le conduit 8 de l'unité de fabrication (par exemple du méthyl-4 pentène-1). Cette charge fraîche est d'abord de préférence indirectement préchauffée par de l'effluent réactionnel chaud (provenant de la conduite 5) à travers l'échangeur de chaleur 9. Elle passe ensuite dans le conduit 10 et va à la rencontre d'une fraction recyclée du fluide de thermorégulation que l'on appellera "charge recyclée" par la suite, cette fraction recyclée étant amenée par le conduit 11.

Le rapport en poids entre la charge recyclée et la charge fraîche est utilement compris entre 1 et 500, de préférence entre 2 et 200, et de manière encore plus préférée entre 5 et 100.

Le mélange ainsi obtenu (charge fraîche + charge recyclée) forme le fluide de thermorégulation (ou fluide caloporteur) du réacteur (1). Ce fluide, qui se trouve en général entre 100 et 200 °C, de préférence aux alentours de 150 °C et à une température réduite d'environ 1, 13, et dans un état tel qu'il peut toujours être véhiculé par une pompe, pénètre par le conduit 2 dans l'intérieur des plaques réfrigérantes creuses (6.3a ou 6.3b) disposées au sein du lit catalytique contenu dans le réacteur (1).

Le fluide de thermorégulation absorbe la chaleur de réaction dégagée au fur et à mesure de la formation du produit de synthèse (par exemple le méthyl-4 pentène-1 obtenu par dimérisation sélective du propylène). Ce fluide sort du réacteur (1) par la conduite 3.

Il est alors séparé en deux portions. Une première portion forme la charge à traiter qui va circuler à travers le lit de catalyseur. Cette charge sera par la suite appelée "charge d'appoint". Elle est envoyée vers le réacteur (1) par la conduite 14. Une deuxième portion du fluide de thermorégulation soutirée par la conduite 3 sera recyclée par la conduite 11 dans les plaques échangeuses de chaleur 6.3a ou 6.3b (en mélange avec de la charge fraîche en provenance de la conduite 10). Cette deuxième portion de fluide de thermorégulation est nommée "charge recyclée".

La "charge recyclée" passe dans le conduit 12 où elle est aspirée par la pompe 13 (de préférence centrifuge) pour être injectée dans la conduite 11 déjà mentionnée.

Selon les caractéristiques de l'invention, le rapport en poids entre la charge recyclée et la charge d'appoint est avantageusement compris entre et 500, de préférence entre 2 et 200 et de manière encore plus préférée entre 5 et 100. Un rapport trop faible interdit un contrôle thermique satisfaisant au sein du lit catalytique un rapport trop élevé demande une section de passage telle que les plaques creuses deviennent trop épaisses et trop encombrantes.

La "charge d'appoint", non reprise par la pompe 13, passe dans le conduit 14 à travers l'échangeur 15 dont le rôle est de réajuster au niveau voulu la température à l'entrée du réacteur 1. Cette charge d'appoint sort de l'échangeur 15 par la conduite 4 et traverse ensuite le lit catalytique contenu dans le réacteur 1. L'effluent réactionnel résultant est évacué dudit réacteur par la conduite 5 pour venir préchauffer la charge fraîche à travers l'échangeur 9. De 9, ledit effluent est utilement dirigé par le conduit 16, sur une unité de distillation et de conditionnement (non représentée sur la Figure).

Lorsqu'il s'agit de dimériser le propylène de manière sélective en méthyl-4 pentène-1, on utilise par exemple un catalyseur à base de carbonate alcalin imprégné de préférence par le métal de la même famille.

Grâce au bon contrôle thermique assuré par le système de la réfrigération à plaques, la conversion a pu être poussée très loin sans que la sélectivité de l'opération en soit affectée.

Ainsi, l'invention est relative à un procédé catalytique de dimérisation ou de codimérisation ou d'oligomérisation d'oléfines effectué habituellement sous pression en présence d'un catalyseur solide, dans une zone de réaction définie par une enceinte de forme sensiblement cyclindrique et dont la section est sensiblement circulaire, ladite enceinte renfermant un lit généralement fixe de catalyseur dans lequel est plongée une pluralité d'espaces internes creux, dans lesquels circule un fluide de thermorégulation, sous une pression sensiblement égale à la pression à laquelle est soumis le mélange réactionnel, procédé dans lequel :

– on introduit une charge fraîche liquide (contenant au moins une oléfine), en mélange avec une charge recyclée définie ci-dessous, dans lesdits espaces internes creux délimités par des parois, le rapport en poids entre la charge recyclée et la charge fraîche étant compris entre 1 et 500, le mélange charge fraîche - charge recyclée formant le fluide de thermorégulation ;

– on soutire le fluide de thermorégulation desdits espaces internes et on sépare ce fluide en deux portions appelées ci-après charge recyclée et charge d'appoint, le rapport en poids entre ladite charge recyclée et ladite charge d'appoint étant compris entre 1 et 500 ;

– on renvoie ladite charge recyclée vers lesdits espaces internes à titre de composant du fluide de thermorégulation ;

– on envoie ladite charge d'appoint dans le lit de catalyseur ;

– on soutire un effluent réactionnel du lit de catalyseur.

Le procédé selon l'invention se caractérise en outre par un cheminement particulier du fluide de thermorégulation (ou liquide caloporteur) dans lesdits espaces internes creux.

Selon l'invention, il y a plusieurs façons de procéder au cheminement de ce fluide, illustrées par les figures 1, 2 et 3 :

– la figure 1 correspond au premier type de cheminement du fluide de thermorégulation ;

– les figures 2 et 3 correspondent au second type de circulation de ce fluide.

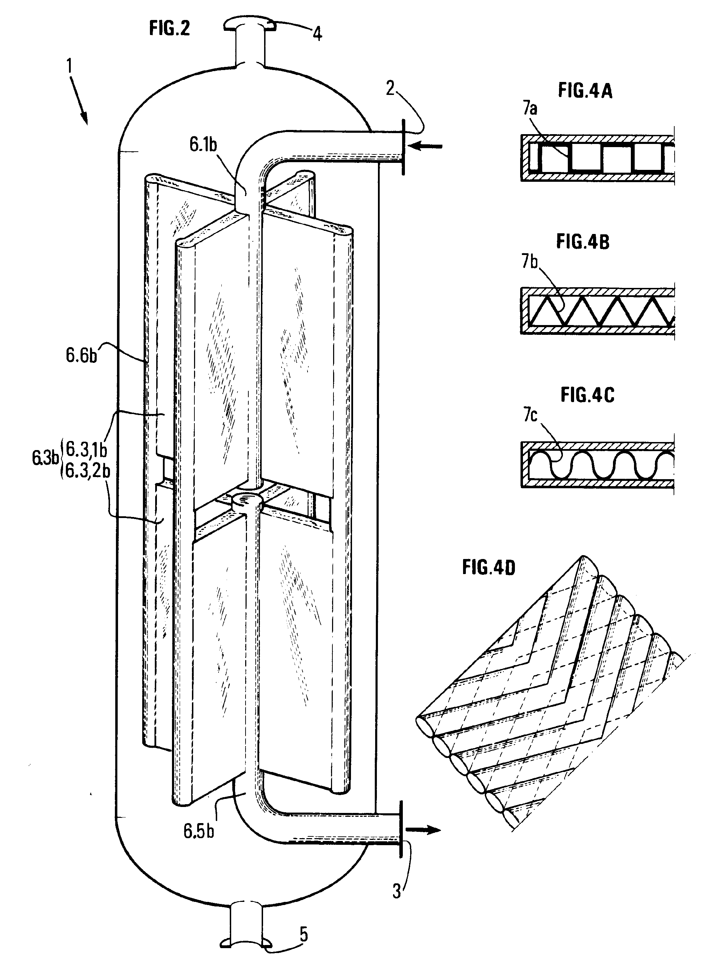

Dans ces figures, les plaques (ou espaces internes creux) sont représentées comme ayant des faces planes. Les figures 4A, 4B, 4C et 4D représentent des plaques selon différents perfectionnements de l'invention.

Dans les deux types de circulation du fluide de thermorégulation, on utilise une zone de réaction ou enceinte (1), de forme sensiblement cylindrique et dont la coupe a une forme sensiblement circulaire, comportant au moins une conduite (2) pour l'introduction dudit fluide de thermorégulation, au moins une conduite (3) pour le soutirage dudit fluide, au moins une conduite (4) pour l'introduction d'une charge dite d'appoint dans la zone de réaction et au moins une conduite (5) pour le soutirage de l'effluent réactionnel de ladite zone de réaction. Le niveau de la conduite d'arrivée 4 et le niveau de la conduite de sortie 5 peuvent être agencés à tout endroit adéquat de l'enceinte (1), par exemple comme indiqué sur les figures 1, 2 et 5.

L'appareil employé dans le premier type de circulation du fluide de thermorégulation renferme en outre (voir figure 1) :

a) au moins un collecteur distributeur central (6.1a), par exemple vertical dont l'axe correspond généralement à l'axe de l'enceinte (1), qui est situé dans la partie supérieure de l'enceinte et est connecté à la conduite 2,

b) une pluralité de collecteurs distributeurs (6.2a) parallèles à l'axe de l'enceinte, ces collecteurs étant connectés individuellement, vers leur sommet, au collecteur distributeur central (6.1 a),

c) au moins un collecteur receveur central (6.5a), par exemple vertical dont l'axe correspond en général à l'axe de l'enceinte, qui est situé dans la partie inférieure de l'enceinte et est connecté à la conduite 3,

d) une pluralité de collecteurs receveurs (6.4a) parallèles à l'axe de l'enceinte, ces collecteurs étant, d'une part, connectés individuellement, vers leur base, au collecteur receveur central (6.5a) et, d'autre part, situés chacun dans le prolongement d'un collecteur distributeur (6.2a) correspondant, la surface de contact entre un collecteur receveur (6.4a) et son collecteur distributeur (6.2a) correspondant étant étanche,

e) une pluralité de collecteurs relieurs (6.6a), chacun d'eux étant, d'une part, parallèle à l'axe de l'enceinte, à un collecteur distributeur (6.2a) et au collecteur receveur (6.4a) correspondant, et, d'autre part, situé dans le même plan défini par ce collecteur distributeur (6.2a), ce collecteur receveur (6.4a) et l'axe de l'enceinte,

f) une pluralité de plaques creuses (espaces internes creux), continues (6.3a) et allongées, destinées à la circulation du fluide de thermorégulation, chaque plaque étant divisée, dans le sens de la largeur, en deux demi-panneaux creux ou demi-espaces internes creux (6.3, 1a et 6.3, 2a), la surface de contact entre ces deux demi-panneaux étant étanche, ces deux demi-panneaux étant ouverts sur un collecteur relieur (6.6a) qui fait communiquer entre eux les deux demi-panneaux (6.3, 1a et 6.3, 2a), le demi-panneau supérieur ou demi-espace supérieur (6.3, 1a) étant ouvert sur un collecteur distributeur (6.2a), le demi-panneau inférieur ou demi-espace inférieur (6.3, 2a) étant ouvert sur le collecteur receveur (6.4a) correspondant.

Selon un perfectionnement, les faces desdites plaques creuses peuvent être constituées par des tôles ondulées dont les ondulations ont au choix l'une des formes suivantes : carrée, rectangulaire, triangulaire, sinusoïdale et en chevrons (voir figure 4D), le but étant de créer une forte turbulence sur l'écoulement du fluide de thermorégulation.

Dans l'appareil utilisé dans le premier type de cheminement du fluide de thermorégulation, lesdites plaques creuses sont sensiblement parallélépipèdiques (6.3a), chaque plaque comportant deux faces larges parallèles délimitant un plan disposé radialement par rapport à l'axe de l'enceinte (1) et quatre faces minces, deux d'entre elles étant parallèles à l'axe de l'enceinte, les deux autres étant perpendiculaires à cet axe chaque plaque est divisée, dans le sens de la largeur, en deux demi-panneaux creux ou demi-espaces internes creux (6.3, 1a et 6.3, 2a), la surface de contact entre ces deux demi-panneaux étant étanche. Ces deux demi-panneaux sont ouverts, sur toute leur face mince parallèle à l'axe de l'enceinte et la plus éloignée de cet axe, sur un collecteur relieur (6.6a) qui fait communiquer entre eux les deux demi-panneaux (6.3, 1a et 6.3, 2a). Le demi-panneau supérieur ou demi-espace supérieur (6.3, 1a) s'ouvre sur toute sa face mince parallèle à l'axe de l'enceinte et la plus proche de cet axe, sur le collecteur receveur (6.4a) correspondant. Lesdites faces minces de chaque plaque creuse peuvent éventuellement être non planes, mais par exemple semi-cylindriques.

Dans la figure 1, donnée à titre d'exemple, on décrira le trajet du fluide de thermorégulation à travers des plaques creuses (espaces internes creux) sensiblement parallélépipèdiques (contenues dans la zone de réaction) :

– on envoie le fluide de thermorégulation, autogène (formé par le(s) composant(s) constituant la charge fraîche (et la charge recyclée par conséquent)), dans une zone de distribution centrale (6.1 a),

– on répartit ce fluide en provenance de la zone de distribution centrale (6.1a) dans des zones distributives (6.2a),

– on fait pénétrer ce fluide, depuis lesdites zones distributives (6.2a), dans lesdits demi-espaces internes creux supérieurs (6.3, 1a) délimités par des parois, chaque demi-espace supérieur (6.3, 1a) ayant sensiblement une forme parallélépipèdique (chaque demi-espace supérieur comportant ainsi deux faces larges parallèles délimitant un plan disposé radialement par rapport à l'axe de la zone de réaction (1) et quatre faces minces, deux d'entre elles étant parallèles à l'axe de la zone de réaction (1), les deux autres étant perpendiculaires à cet axe), ledit fluide pénètrant dans lesdits demi-espaces supérieurs (6.3, 1a) par leur face mince parallèle à l'axe de la zone de réaction définie par une enceinte (1) de forme sensiblement cylindrique, cette face étant la plus proche de cet axe,

– on fait circuler ledit fluide à l'intérieur desdits demi-espaces internes creux supérieurs (6.3, 1a) sous la forme d'une nappe,

– on évacue ledit fluide desdits demi-espaces supérieurs (6.3, 1a), par leur face mince parallèle à l'axe de la zone de réaction (1) et la plus éloignée de cet axe, dans des zones relieuses (6.6a) qui relient chacune un demi-espace supérieur (6.3, 1a) avec un demi-espace interne creux inférieur (6.3, 2a) situé dans le prolongement dudit demi-espace supérieur (6.3, 1a), chaque demi-espace inférieur (6.3, 2a) étant délimité par des parois et ayant sensiblement une forme parallélépipèdique qui est définie comme pour un demi-espace supérieur (6.3, 1a),

– on fait pénétrer ledit fluide, depuis lesdites zones relieuses (6.6a), dans lesdits demi-espaces internes creux inférieurs (6.3, 2a) par leur face mince parallèle à l'axe de la zone de réaction (1) et la plus éloignée de cet axe,

– on fait circuler ledit fluide à l'intérieur desdits demi-espaces internes creux inférieurs (6.3, 2a) sous la forme d'une nappe,

– on évacue ledit fluide de thermorégulation desdits demi-espaces inférieurs (6.3, 2a), par leur face mince parallèle à l'axe de la zone de réaction (1) et la plus proche de cet axe, dans des zones collectrices (6.4a) qui sont reliées à une zone de collecte centrale (6.5a) par laquelle on soutire ensuite ledit fluide.

L'appareil utilisé dans le deuxième type de circulation du fluide de thermorégulation renferme en outre (voir figure 2) :

a) au moins un collecteur distributeur central (6.1b), par exemple vertical, dont l'axe correspond en général à l'axe de l'enceinte (1), qui est situé au-dessus d'un collecteur receveur central (6.5b) défini ci-dessous et qui est connecté à la conduite 2,

b) une pluralité de collecteurs relieurs (6.6b) parallèles à l'axe de l'enceinte,

c) au moins un collecteur receveur central (6.5b), par exemple vertical dont l'axe correspond généralement à l'axe de l'enceinte, qui est situé en-dessous du collecteur distributeur central (6.1b) et qui est connecté à la conduite 3,

d) une pluralité de plaques creuses (ou espaces internes creux), continues (6.3b) et allongées, destinées à la circulation du fluide de thermorégulation, lesdites plaques étant associées deux à deux, chaque association comportant deux séries de plaques, une première série de la plaques (6.3, 1b) se situant au-dessus de la deuxième série, chacune des plaques de cette première série (6.3, 1b) étant ouverte sur un collecteur relieur (6.6b) et sur le collecteur distributeur central (6.1b), une deuxième série de plaques (6.3, 2b) se situant en-dessous de la première série de plaques (6.3, 1b), chacune des plaques de cette deuxième série (6.3, 2b) étant située dans le prolongement d'une plaque de la première série (6.3, 1b) (les plaques de la première série n'étant pas adjacentes à celles de la deuxième série), étant ouverte sur un collecteur relieur (6.6b) qui fait communiquer entre elles chaque plaque de cette deuxième série avec une plaque de la première série située dans son prolongement, et étant ouverte sur le collecteur receveur central (6.5b).

Selon un perfectionnement, les faces desdites plaques creuses peuvent être constituées par des tôles ondulées dont les ondulations ont au choix l'une des formes suivantes : carrée, rectangulaire, triangulaire, sinusoïdale ou en chevrons (voir figure 4D), le but étant de créer également une forte turbulence sur l'écoulement du fluide de thermorégulation.

Dans l'appareil employé dans le deuxième type de cheminement du fluide de thermorégulation, lesdites plaques creuses sont sensiblement parallélépipèdiques, chaque plaque comportant deux faces larges parallèles délimitant un plan disposé radialement par rapport à l'axe de l'enceinte (1) et quatre faces minces, deux d'entre elles étant parallèles à l'axe de l'enceinte, les deux autres étant perpendiculaires à cet axe. Lesdites plaques sont associées deux à deux, chaque association comportant deux séries de plaques, une première série de plaques (6.3, 1b) se situant au-dessus de la deuxième série (6.3, 2b). Chacune des plaques de cette première série (6.3, 1b) est ouverte, sur toute sa face mince parallèle à l'axe de l'enceinte et la plus éloignée de cet axe, sur un collecteur relieur (6.6b) et ouverte, sur toute sa face mince parallèle à l'axe de l'enceinte et la plus proche de cet axe, sur le collecteur distributeur central (6.1b). La deuxième série de plaques (6.3, 2b) se situe en-dessous de la première série de plaques (6.3, 1b), chacune des plaques de cette deuxième série (6.3, 2b) étant située dans le prolongement d'une plaque de la première série (6.3, 1b) (les plaques de la première série n'étant pas adjacentes à celles de la deuxième série) ; chacune des plaques de la deuxième série (6.3, 2b) est ouverte d'une part, sur toute sa face mince parallèle à l'axe de l'enceinte (1) et la plus éloignée de cet axe, sur un collecteur relieur (6.6b) qui fait communiquer entre elles chaque plaque de cette deuxième série avec une plaque de la première série située dans son prolongement, et d'autre part, sur toute sa face mince parallèle à l'axe de l'enceinte et la plus proche de cet axe, sur le collecteur receveur central (6.5b). Lesdites faces minces de chaque plaque creuse peuvent éventuellement être non planes, mais par exemple semi-cylindriques.

Dans la figure 2, donnée à titre d'exemple, on décrira le trajet du fluide de thermorégulation à travers des plaques creuses (ou espaces internes creux) sensiblement parallélépipèdiques (contenues dans la zone de réaction (1)) :

– on introduit le fluide de thermorégulation (autogène) dans une zone de distribution centrale (6.1b),

– on fait pénétrer ledit fluide, depuis ladite zone de distribution centrale (6.1b), dans une première série d'espaces internes creux (6.3, 1b) délimités par des parois et de forme sensiblement parallélépipèdique (chaque espace parallélépipèdique de la première série comportant ainsi deux faces larges parallèles délimitant un plan disposé radialement par rapport à l'axe de la zone de réaction (1) et la quatre faces minces, deux d'entre elles étant parallèles à l'axe de la zone de réaction (1), les deux autres étant perpendiculaires à cet axe), ledit fluide pénétrant dans lesdits espaces internes creux de la première série (6.3, 1b) par leur face mince parallèle à l'axe de la zone de réaction (1) et la plus proche de cet axe,

– on fait cheminer ledit fluide à l'intérieur desdits espaces internes creux de la première série (6.3, 1b) sous la forme d'une nappe,

– on évacue ledit fluide desdits espaces internes creux de la première série (6.3, 1b), par leur face mince parallèle à l'axe de la zone de réaction (1) et la plus éloignée de cet axe, dans des zones relieuses (6.6b) qui font chacune communiquer entre eux un espace interne creux de la première série (6.3, 1b) avec un espace interne creux d'une deuxième série (6.3, 2b) situé dans son prolongement, chaque espace de la deuxième série (6.3, 2b) étant délimité par des parois et ayant une forme sensiblement parallélépipèdique qui est définie comme pour un espace de la première série (6.3, 1b),

– on fait pénétrer ledit fluide, depuis lesdites zones relieuses (6.6b), dans lesdits espaces internes creux de la deuxième série (6.3, 2b), par leur face mince parallèle à l'axe de la zone de réaction et la plus éloignée de cet axe,

– on fait circuler ledit fluide à l'intérieur desdits espaces internes creux de la deuxième série (6.3, 2b) sous la forme d'une nappe,

– on évacue ledit fluide de thermorégulation desdits espaces internes creux de la deuxième série (6.3, 2b), par leur face mince parallèle à l'axe de la zone de réaction (1) et la plus proche de cet axe, dans une zone de collecte centrale (6.5b) par laquelle on soutire ensuite ledit fluide.

Selon un perfectionnement, l'appareil du deuxième type peut renfermer plusieurs associations de deux séries de plaques (ou espaces internes), lesdites associations étant empilées le long de l'axe de l'enceinte (voir figure 3). Sur la figure 3, on a arbitrairement représenté deux associations de deux séries de plaques chacune, mais le nombre de ces associations peut être plus élevée ; sur cette figure 3, le fluide de thermorégulation suit le trajet matérialisé par les flèches F, de proche en proche à travers deux associations de deux séries de plaques ; ce fluide fait ainsi successivement deux fois le trajet décrit par la figure 2. Quand le nombre d'associations de deux séries de plaques est égal à n, avec n supérieur à deux, alors le fluide de thermorégulation fait successivement n fois le trajet décrit pour la figure 2.

L'avantage d'un fluide de thermorégulation autogène est, d'une part, qu'il n'y a pas de différence de pression entre l'intérieur et l'extérieur des plaques (à part celle créée par les pertes de charge dues à la circulation des différents fluides) et, d'autre part, qu'en cas de fuite, il n'y a pas de danger de pollution du système catalytique.

Selon une variante de l'invention, lesdites plaques creuses peuvent, éventuellement, avoir des largeurs différentes, ce qui permet de maintenir un rapport minimum entre le volume de l'enceinte et la surface d'échange, tout en évitant d'avoir une trop grande distance entre un point quelconque de 'enceinte et la plaque la plus proche.

A noter qu'on peut aménager dans des plaques creuses sensiblement parallélépipèdiques (voir figures 4A, 4B et 4C) des canaux adjacents au moyen de tôles ondulées, les sections desdits canaux ayant au choix l'une des formes suivantes : carrée, rectangulaire (7a), triangulaire (7b) et sinusoïdale (7c), ces canaux reliant entre elles les deux faces minces parallèles à l'axe de l'enceinte d'une même plaque : d'une part, la présence de ces canaux adjacents assure la solidité des plaques creuses qui peuvent atteindre et dépasser, par exemple, dix mètres de hauteur et d'autre part, elle évite la la formation de zones mortes (c'est-à-dire de zones non traversées par le fluide de thermorégulation), zones mortes qui pourraient se former du fait de 'écoulement en nappe du fluide de thermorégulation à l'intérieur des plaques.

L'assemblage des tôles peut être réalisé soit par soudure, soit beaucoup plus économiquement par brasure, soit par points, soit par immersion dans un bain, ou toute autre technique adéquate.

Les tôles éventuellement utilisées dans les divers modes de réalisation de l'invention ont, généralement, moins de 10 millimètres d'épaisseur, de préférence moins de 3 millimètres d'épaisseur.

Sur les figures, l'enceinte (1) est représentée en position sensiblement verticale : les circulations du fluide de thermorégulation et de la charge d'appoint peuvent se faire de haut en bas, comme décrit précédemment, mais également de bas en haut (et donc aussi à contrecourant).

Chacune des figures 1, 2 et 3 représente un(e) réacteur (enceinte) axial(e) dans lequel (laquelle) les réactifs traversent le lit de catalyseur de façon parallèle à l'axe du réacteur.

L'invention peut s'appliquer également à un réacteur radial comportant un panier perméable de la forme d'un anneau cylindrique, par exemple, délimité par deux cylindres coaxiaux, dans lequel sont disposés le catalyseur et les plaques creuses et où les réactifs traversent le lit perpendiculairement à 'axe du réacteur.

EXEMPLE 1 (selon l'invention)

Dans un réacteur cylindrique vertical de 0,5 mètre de diamètre, équipé d'un système de thermorégulation à plaques creuses conforme aux figures 1 et 4A, on dispose du catalyseur obtenu par dépôt de 3,5 % en poids de sodium sur des écailles de carbonate de potassium lié par 1,5 % de graphite, et préalablement activé à 230 °C pendant 3 heures.

Puis on remplit, sous une pression de 9 MPa, l'unité représentée selon la figure 3 (par le conduit 8) avec du propane et on assure au moyen de la pompe 13 une recirculation de 300 m³/h.

Au moyen du réchauffeur extérieur à vapeur (15), on élève progressivement la température du propane. Quand cette température atteint 150 °C environ, on introduit du propylène dans l'unité (par le conduit 8), tout en purgeant le propane par le conduit 16.

Au bout de quelques heures, il s'établit un régime stationnaire avec un débit de propylène de 5 m³/h, une conversion du propylène de 28,9 % et une sélectivité en méthyl-4 pentène-1 de 89,8 %. Ce régime a pu être maintenu pendant plusieurs centaines d'heures sans variation sensible de la conversion du propylène et de la sélectivité en méthyl-4 pentène-1.

EXEMPLE 2 (selon l'invention)

On opère dans des conditions identiques à celles de l'exemple 1, le système de thermorégulation à plaques creuses utilisé étant conforme aux figures 2 et 4C.

Au bout de quelques heures de fonctionnement, il s'établit un régime stationnaire avec un débit de propylène de 5 m³/h, une conversion du propylène de 29,1 % et une sélectivité en méthyl-4 pentène-1 de 89,7 %. Ce régime a pu être maintenu pendant plusieurs centaines d'heures sans variation sensible de cette conversion et de cette sélectivité.

EXEMPLE 3 (comparatif)

On utilise le même catalyseur que dans l'exemple 1, que l'on dispose dans le même réacteur d'où on a enlevé le système de thermorégulation selon l'invention, le débit horaire de propylène étant identique.

La température d'alimentation du réacteur en propylène est de 130 °C. La température de l'effluent réactionnel à la sortie du réacteur est de 190 °C.

On note que la conversion du propylène ne dépasse pas 15,0 % et la sélectivité en méthyl-4 pentène-1 n'atteint que 65,1 %, la majeure partie des sous-produits de la réaction étant formée par du méthyl-4 pentène-2. On constate de plus qu'après une cinquantaine d'heures, le catalyseur a perdu une grande partie de son activité.

**Revendications**

1. Procédé catalytique de dimérisation ou de codimérisation ou d'oligomérisation d'oléfines effectué sous pression en présence d'un catalyseur solide, dans une zone de réaction définie par une enceinte de forme sensiblement cylindrique et dont la section est sensiblement circulaire, ladite enceinte renfermant un lit de catalyseur dans lequel est plongée une pluralité d'espaces internes creux, dans lesquels circule un fluide de thermorégulation, sous une pression sensiblement égale à la pression à laquelle est soumis le mélange réactionnel chaque espace interne creux étant divisé dans le sens de la largeur en un demi-espace interne creux supérieur et un demi-espace interne creux inférieur, procédé dans lequel :

   – on introduit une charge fraîche liquide contenant au moins une oléfine, en mélange avec une charge recyclée définie ci-dessous, dans lesdits espaces internes creux délimités par des parois, le rapport en poids entre la charge recyclée et la charge fraîche étant compris entre 1 et 500, le mélange charge fraîche - charge recyclée formant le fluide de thermorégulation,

– on soutire le fluide de thermorégulation desdits espaces internes et on sépare ce fluide en deux portions appelées ci-après charge recyclée et charge d'appoint, le rapport en poids entre ladite charge recyclée et ladite charge d'appoint étant compris entre 1 et 500,

– on renvoie ladite charge recyclée vers lesdits espaces internes à titre de composant du fluide de thermorégulation,

– on envoie ladite charge d'appoint dans le lit de catalyseur,

– on soutire un effluent réactionnel du lit de catalyseur, procédé dans lequel en outre :

. on envoie le fluide de thermorégulation dans une zone de distribution centrale,

. on répartit ce fluide en provenance de la zone de distribution centrale dans des zones distributives,

. on fait pénétrer ce fluide, depuis lesdites zones distributives, dans lesdits demi-espaces internes creux supérieurs délimités par des parois, chaque demi-espace supérieur ayant sensiblement une forme parallélépipèdique, ledit fluide pénétrant dans lesdits demi-espaces internes supérieurs creux par leur face mince parallèle à l'axe de la zone de réaction et la plus proche de cet axe,

. on fait circuler ledit fluide à l'intérieur desdits demi-espaces internes creux supérieurs sous la forme d'une nappe,

. on évacue ledit fluide desdits demi-espaces supérieurs, par leur face mince parallèle à l'axe de la zone de réaction et la plus éloignée de cet axe, dans des zones relieuses qui relient chacune un demi-espace supérieur avec un demi-espace interne creux inférieur situé dans le prolongement dudit demi-espace supérieur, chaque demi-espace inférieur étant délimité par des parois et ayant sensiblement une forme parallélépipèdique,

. on fait pénétrer ledit fluide, depuis lesdites zones relieuses, dans lesdits demi-espaces internes creux inférieurs, par leur face mince parallèle à l'axe de la zone de réaction et la plus éloignée de cet axe,

. on fait circuler ledit fluide à l'intérieur desdits demi-espaces internes creux inférieurs sous la forme d'une nappe,

. on évacue ledit fluide de thermorégulation desdits demi-espaces inférieurs, par leur face mince parallèle à l'axe de la zone de réaction et la plus proche de cet axe, dans des zones collectrices qui sont reliées à une zone de collecte centrale par laquelle on soutire ensuite ledit fluide.

2. Procédé catalytique de dimérisation ou de codimérisation ou d'oligomérisation d'oléfines effectué sous pression en présence d'un la catalyseur solide, dans une zone de réaction définie par une enceinte de forme sensiblement cylindrique et dont la section est sensiblement circulaire, ladite enceinte renfermant un lit de catalyseur dans lequel est plongée une pluralité d'espaces internes creux, dans lesquels circule un fluide de thermorégulation, sous une pression sensiblement égale à la pression à laquelle est soumis le mélange réactionnel, procédé dans lequel :

– on introduit une charge fraîche liquide contenant au moins une oléfine, en mélange avec une charge recyclée définie ci-dessous, dans lesdits espaces internes creux délimités par des parois, le rapport en poids entre la charge recyclée et la charge fraîche étant compris entre 1 et 500, le mélange charge fraîche - charge recyclée formant le fluide de thermorégulation,

– on soutire le fluide de thermorégulation desdits espaces internes et on sépare ce fluide en deux portions appelées ci-après charge recyclée et charge d'appoint, le rapport en poids entre ladite charge recyclée et ladite charge d'appoint étant compris entre 1 et 500,

– on renvoie ladite charge recyclée vers lesdits espaces internes à titre de composant du fluide de thermorégulation,

– on envoie ladite charge d'appoint dans le lit de catalyseur,

– on soutire un effluent réactionnel du lit de catalyseur, procédé dans lequel en outre :

. on introduit le fluide de thermorégulation dans une zone de distribution centrale,

. on fait pénètrer ledit fluide, depuis ladite zone de distribution centrale, dans une première série d'espaces internes creux délimités par des parois et de forme sensiblement parallélépipèdique, ledit fluide pénétrant dans lesdits espaces internes creux de la première série par leur face mince parallèle à l'axe de la zone de réaction et la plus proche de cet axe,

. on fait cheminer ledit fluide à l'intérieur desdits espaces internes creux de la première série sous la forme d'une nappe,

. on évacue ledit fluide desdits espaces internes creux de la première série, par leur face mince parallèle à l'axe de la zone de réaction et la plus éloignée de cet axe, dans des zones relieuses qui font chacune communiquer entre eux un espace interne creux de la première série avec un espace interne creux d'une deuxième série situé dans son prolongement, chaque espace de la deuxième série étant délimité par des parois et ayant une forme sensiblement parallélépipèdique,

. on fait pénètrer ledit fluide, depuis lesdites zones relieuses, dans lesdits espaces internes creux de la deuxième série par leur face mince parallèle à l'axe de la zone de réaction et la plus éloignée de cet axe,

. on fait circuler ledit fluide à l'intérieur desdits espaces internes creux de la deuxième série sous la forme d'une nappe,

. on évacue ledit fluide de thermorégulation desdits espaces internes creux de la deuxième série, par leur face mince parallèle à l'axe de la zone de réaction et la plus proche de cet axe, dans une zone de collecte centrale par laquelle on soutire ensuite ledit fluide.

3. Procédé selon la revendication 2 caractérisé en ce qu'on utilise plusieurs associations de deux séries d'espaces internes creux, lesdites associations étant empilées le long de l'axe de la zone de réaction.

4. Procédé selon l'une des revendications 1 à 3 dans lequel la charge fraîche est préchauffée par contact indirect avec 'effluent réactionnel.

5. Procédé selon l'une des revendications 1 à 4 dans lequel le rapport en poids entre ladite charge recyclée et ladite charge fraîche est compris entre 2 et 200.

6. Procédé selon l'une des revencications 1 à 5 dans lequel le rapport en poids entre ladite charge recyclée et ladite charge d'appoint est compris entre 2 et 200.

7. Procédé selon l'une des revendications 1 à 6 appliqué à la dimérisation sélective du propylène en méthyl-4 pentène-1.

## Patentansprüche

1. Katalytisches Verfahren zum Dimerisieren oder Codimerisieren oder Oligomerisieren von Olefinen, unter Druck durchgeführt, in Anwesenheit eines festen Katalysators in einer durch einen umschlossenen Raum von im wesentlichen zylindrischer Form definierten Reaktionszone und dessen Querschnitt im wesentlichen kreisförmig ist, wobei dieser umschlossene Raum ein Katalysatorbett umschließt, in welches eine Vielzahl innen hohler Räume taucht, in denen ein Wärmeregulierungsfluid unter einem Druck zirkuliert, der im wesentlichen gleich dem Druck ist, dem das Reaktionsgemisch ausgesetzt ist, wobei jeder innen hohle Raum in Richtung der Breite in einen oberen innen hohlen Halbraum und einen unteren innen hohlen Halbraum unterteilt ist, Verfahren bei dem:

– man eine frische flüssige wenigstens ein Olefin enthaltende Charge im Gemisch mit einer unten definierten rezyklisierten Charge in diese durch Wände begrenzten inneren hohlen Räume einführt, wobei das Gewichtsverhältnis zwischen der rezyklisierten Charge und der frischen Charge zwischen 1 und 500 beträgt und wobei das Gemisch frische Charge - rezyklisierte Charge das Wärmeregulierungsfluid bildet,

– man das Wärmeregulierungsfluid aus diesen Innenräumen abzieht und man das Fluid in zwei anschließend rezyklisierte Charge und Zuführcharge genannte Teile trennt, wobei das Gewichtsverhältnis zwischen dieser rezyklisierten Charge und dieser Zuführcharge zwischen 1 und 500 beträgt,

– man diese rezyklisierte Charge gegen die Innenräume als Wärmeregelungsfluidkomponente zurückschickt,

– man diese Zuführcharge in das Katalysatorbett gibt,

– man einen Reaktionsabstrom aus dem Katalysatorbett abzieht, wobei nach diesem Verfahren im übrigen:

. man das Wärmeregulierungsfluid in eine allgemeine Verteilerzone schickt,

. man dieses aus der zentralen Verteilerzone stammende Fluid in verteilende Zonen aufteilt,

. man dieses Fluid aus diesen verteilenden Zonen in diese oberen innen hohlen Halbräume, die durch Wandungen begrenzt sind, eindringen läßt, wobei jeder obere Halbraum im wesentlichen eine parallelepipedförmige Gestalt hat und dieses Fluid in diese oberen innen hohlen Halbräume über ihre Schmalseite parallel zur Achse der Reaktionszone und die dieser Achse am nächsten liegt, eindringt,

. man dieses Fluid im Inneren dieser innen hohlen oberen Halbräume in Form einer Bahn strömen läßt,

. man dieses Fluid aus diesen oberen Halbräumen über ihre Schmalseite parallel zur Achse der Reaktionszone, die von dieser Achse am weitesten entfernt liegt, in Verbindungszonen evakuiert, welche je einen oberen Halbraum mit einem unteren innen hohlen Raum verbinden, der in der Verlängerung dieses oberen Halbraums angeordnet ist, wobei jeder untere Halbraum durch Wandungen begrenzt ist und im wesentlichen von parallelepipedförmiger Gestalt ist,

. man dieses Fluid aus diesen Verbindungszonen in diese unteren innen hohlen Räume über ihre Schmalseite parallel zur Achse der Reaktionszone, die von dieser Achse am weitesten entfernt liegt, eindringen läßt,

. man dieses Fluid im Inneren dieser innen hohlen unteren Halbräume in Form einer Bahn strömen läßt und

. man dieses Wärmeregulierungsfluid aus diesen unteren Hohlräumen über ihre Schmalseite parallel zur Achse der Reaktionszone und die dieser Achse am weitesten benachbart ist, in Sammlerzonen abzieht, die mit einer zentralen Sammlerzone verbunden sind, über die man dann dieses Fluid abzieht.

2. Katalytisches Verfahren zur Dimerisierung oder Codimerisierung oder Oligomerisieren von Olefinen, unter Druck durchgeführt, in Anwesenheit eines festen Katalysators in einer durch einen umschlossenen Raum von im wesentlichen zylindrischer Form definierten Reaktionszone und dessen Querschnitt im wesentlichen kreisförmig ist, wobei dieser umschlossene Raum ein Katalysatorbett umschließt, in welches eine Vielzahl innen hohler Räume taucht, in denen ein Wärmeregulierungsfluid unter einem Druck zirkuliert, der im wesentlichen gleich dem Druck ist, dem das Reaktionsgemisch ausgesetzt ist, Verfahren bei dem:

– man eine frische flüssige wenigstens ein Olefin enthaltende Charge im Gemisch mit einer unten definierten rezyklisierten Charge in diese durch Wände begrenzten inneren hohlen Räume einführt, wobei das Gewichtsverhältnis zwischen der rezyklisierten Charge und der frischen Charge zwischen 1 und 500 beträgt und wobei das Gemisch frische Charge - rezyklisierte Charge das Wärmeregulierungsfluid bildet,

– man das Wärmeregulierungsfluid aus diesen Innenräumen abzieht und man das Fluid in zwei anschließend rezyklisierte Charge und Zuführcharge genannte Teile trennt, wobei das Gewichtsverhältnis zwischen dieser rezyklisierten Charge und dieser Zuführcharge zwischen 1 und 500 beträgt,

– man diese rezyklisierte Charge gegen die Innenräume als Wärmeregelungsfluidkomponente zurückschickt,

– man diese Zuführcharge in das Katalysatorbett gibt,

– man einen Reaktionsabstrom aus dem Katalysatorbett abzieht, wobei nach diesem Verfahren im übrigen:

. man das Wärmeregulierungsfluid in eine zentrale Verteilerzone einführt,

. man dieses Fluid, ausgehend von der zentralen Verteilerzone in eine erste Reihe von innen hohlen Räumen eindringen läßt, die durch Wandungen begrenzt sind und von einer im wesentlichen parallelepipedförmigen Gestalt sind, wobei das Fluid in diese innen hohlen Räume aus der ersten Reihe über ihre Schmalseite parallel zur Achse der Reaktionszone, die dieser Achse am weitesten benachbart ist, eindringt,

. man dieses Fluid im Inneren dieser innen hohlen Räume aus der ersten Reihe in Form einer Bahn strömen läßt,

. man dieses Fluid aus diesen innen hohlen Räumen der ersten Reihe über ihre Schmalseite parallel zur Achse der Reaktionszone, die am weitesten von dieser Achse entfernt ist, in Verbindungszonen evakuiert, die miteinander einen innen hohlen Raum der ersten Reihe mit einem Innenhohlraum einer zweiten Reihe kommunizieren lassen, der in seiner Verlängerung angeordnet ist, wobei jeder Raum der zweiten Reihe begrenzt ist durch Wandungen und eine im wesentlichen epipedförmige Gestalt hat,

. man dieses Fluid aus diesen Verbindungszonen in diese innen hohlen Räume der zweiten Reihe über ihre Schmalseite parallel zur Achse der Reaktionszone und die von dieser Achse am weitesten entfernt ist, eindringen läßt,

. man dieses Fluid im Inneren dieser innen hohlen Räume aus der zweiten Reihe in Form einer Bahn flächig strömen läßt und

. man dieses Wärmeregulierungsfluid aus diesen innen hohlen Räumen der zweiten Reihe über ihre Schmalseite, parallel zur Achse der Reaktionszone, und die dieser Achse am weitesten benachbart ist, in eine zentrale Sammlerzone evakuiert, über die man dann dieses Fluid abzieht.

3. Verfahren nach Anspruch 2, dadurch gekennzeichnet, daß man mehrere Zuordnungen von zwei Reihen hohler Innenräume verwendet, wobei diese Zuordnungen längs der Achse der Reaktionszone gestapelt sind.

4. Verfahren nach einem der Ansprüche 1 bis 3, bei dem die frische Charge im indirekten Kontakt mit dem Reaktionsabstrom vorgewärmt wird.

5. Verfahren nach einem der Ansprüche 1 bis 4, bei dem das Gewichtsverhältnis zwischen dieser rezyklisierten Charge und dieser frischen Charge zwischen 2 und 200 beträgt.

6. Verfahren nach einem der Ansprüche 1 bis 5, bei dem das Gewichtsverhältnis zwischen dieser rezyklisierten Charge und dieser Zuführcharge zwischen 2 und 200 beträgt.

7. Verfahren nach einem der Ansprüche 1 bis 6, angewendet auf die selektive Dimerisierung des Propylens in 4-Methyl-1-penten.

## Claims

1. A catalytic method for dimerizing or codimerizing or oligomerizing olefins, carried out under pressure, in the presence of a solid catalyst, in a reaction zone defined by a substantially cylindrical enclosure whose section is substantially circular, said enclosure containing a catalyst bed in which a plurality of hollow internal spaces is disposed, through which flows a thermoregulation fluid, at a pressure substantially equal to the pressure to which the reaction mixture is subjected, each hollow internal space being divided in the width direction into an upper hollow internal semi-space and a lower hollow internal semi-space, in which method :

– a fresh liquid charge containing at least one olefin, mixed with a recycled charge defined below, is introduced into said hollow internal spaces defined by walls, the weight ratio between the recycled charge and the fresh charge being between 1 and 500, the fresh charge-recycled charge mixture forming the thermoregulation fluid;

– the thermoregulation fluid is drawn off from said internal spaces and this fluid is separated into two portions called hereafter recycled charge and make-up charge, the weight ratio between said recycled charge and said make-up charge being between 1 and 500;

– said recycled charge is fed into said hollow internal spaces as thermoregulation fluid component;

– said make-up charge is fed into said catalyst bed;

– a reaction effluent is drawn off from said catalyst bed, in which method in addition :

    . the thermoregulation fluid is fed into a central distributing zone,

    . said fluid from the central distributing zone is divided up into distributing zones,

    . said fluid is caused to penetrate, from said distributing zones into said upper hollow internal semi-spaces, defined by walls, each upper semi-space having a substantially parallelepipedic shape , said fluid penetrating into said hollow upper internal semi-spaces through their thin face parallel to the axis of the reaction zone and the closest to this axis,

    . said fluid is caused to flow inside said upper hollow internal semi-spaces in the form of a sheet,

    . said fluid is discharged, from said upper semi-spaces, through their thin face parallel to the axis of the reaction zone and the furthest away from this axis, into connecting zones which each connect an upper semi-space with a lower hollow internal semi-space situated in the extension of said upper semi-space, each lower semi-space being defined by walls and having a substantially parallelepipedic shape,

    . said fluid is caused to penetrate, from said connecting zones, into said lower hollow internal semi-spaces, through their thin face parallel to the axis of the reaction zone and the furthest from this axis,

    . said fluid is caused to flow inside said lower hollow internal semi-space in the form of a sheet,

    . said thermoregulation fluid is discharged, from said lower semi-spaces, through their thin face parallel to the axis of the reaction zone and the closest to this axis, into collecting zones which are connected to a central collecting zone from which said fluid is then drawn off.

2. A catalytic method for dimerizing or codimerizing or oligomerizing olefins, carried out under pressure, in the presence of a solid catalyst, in a reaction zone defined by a substantially cylindrical enclosure whose section is substantially circular, said enclosure containing a catalyst bed in which a plurality of hollow internal spaces is disclosed, through which flows a thermoregulation fluid, at a pressure substantially equal to the pressure to which the reaction mixture is subjected, in which method :

– a fresh liquid charge containing at least one olefin, mixed with a recycled charge defined below, is introduced into said hollow internal spaces defined by walls, the weight ratio between the recycled charge and the fresh charge being between 1 and 500, the fresh charge-recycled charge mixture forming the thermoregulation fluid;

– the thermoregulation fluid is drawn off from said internal spaces and this fluid is separated into two portions called hereafter recycled charge and make-up charge, the weight ratio between said recycled charge and said make-up charge being between 1 and 500;

– said recycled charge is fed into said hollow internal spaces as thermoregulation fluid component;

– said make-up charge is fed into said catalyst bed;

– a reaction effluent is drawn off from said catalyst bed, in which method in addition :

    . the thermoregulation fluid is fed into a central distributirg zone ,

    . said fluid is caused to penetrate, from said central distributing zone, into a first series of hollow internal spaces defined by walls and of a substantially parallelepipedic shape, said fluid penetrating into said hollow internal spaces of the first series through their thin face parallel to the axis of the reaction zone and the closest to this axis,

    . said fluid is caused to flow inside said upper hollow internal spaces of the first series in the form of a sheet,

    . said fluid is discharged, from said hollow internal spaces of the first series, through their thin face parallel to the axis of the reaction zone and the furthest away from this axis, into connecting zones which each cause a hollow internal space of the first series to communicate with a hollow internal space of a second series situated in its extension, each space of the second series being defined by walls and having a substantially parallelepipedic shape ,

. said fluid is caused to penetrate, from said connecting zones, into said hollow internal spaces of the second series through their thin face parallel to the axis of the reaction zone and the furthest from this axis,

. said fluid is caused to flow inside said hollow internal spaces of the second series in the form of a sheet,

. said thermoregulation fluid is discharged, from said hollow internal spaces of the second series, through their thin face parallel to the axis of the reaction zone and the closest to this axis, into a central collecting zone from which from which said fluid is then drawn off.

3. A method according to claim 2 wherein several associations of two series of hollow internal spaces are used, said associations being stacked along the axis of the reaction zone.

4. A method according to one of claims 1 to 3 wherein the fresh charge is pre-heated by indirect contact with the reaction effluent.

5. A method according to one of claims 1 to 4 wherein the weight ratio between said recycled charge and said fresh charge is between 2 and 200.

6. A method according to one of claims 1 to 5 wherein the weight ratio between said recycled charge and said make-up charge is between 2 and 200.

7. A method according to one of claims 1 to 6 applied to the selective dimerization of propylene into methyl-4 pentene-1.

# FIG.1

# FIG.3

**FIG.2**

**FIG.4A**

**FIG.4B**

**FIG.4C**

**FIG.4D**

**FIG.5**